# EUROPEAN PATENT SPECIFICATION

(11) **EP 3 578 161 B1**
(45) Date of publication and mention of the grant of the patent: **12.11.2025**
(21) Application number: 18748710.3
(22) Date of filing: 16.01.2018
(51) Int. Cl.: A61K 8/34, A61K 8/06, A61Q 19/00

(54) **METHOD FOR IMPROVING STORAGE STABILITY OF COSMETIC**
VERFAHREN ZUR VERBESSERUNG DER LAGERSTABILITÄT VON KOSMETIKA
MÉTHODE D'AMÉLIORATION DE LA STABILITÉ AU STOCKAGE D'UN PRODUIT COSMÉTIQUE

(30) Priority: 03.02.2017 JP 2017018349
(43) Date of publication of application: 11.12.2019
(73) Proprietor: Adeka Corporation, Tokyo 116-8554 (JP)
(72) Inventor: INOUE, Takahiro, Tokyo 116-8554 (JP); TACHIYANAGI, Satomi, Tokyo 116-8554 (JP); SUZUKI, Hiroshi, Tokyo 116-8554 (JP)
(74) Representative: Kador & Partner Part mbB
(86) International application number: PCT/JP2018/000991
(87) International publication number: WO 2018/142917

(56) References cited:
- EP-A1- 3 100 715
- EP-A1- 3 111 918
- WO-A1-2015/125392
- JP-A- 2003 342 146
- JP-A- 2007 291 049
- JP-A- 2011 173 808
- JP-A- 2013 095 754
- JP-A- H11 322 591
- ADEKA: "ADEKA NOL CHG Light yellow liquid Arbitrarily miscible Antibacterial Properties", 9 June 2016 (2016-06-09), XP055746982, Retrieved from the Internet <URL:https://www.adeka.co.jp/en/chemical/catalog/pdf/adeka_CHG.pdf> [retrieved on 20201104]
- N KONAT� ET AL: "Sustainably Sourced Pentylene Glycol - a Green All-Rounder", S�FW JOURNAL: SEIFEN, �LE, FETTE, WACHSE, vol. 10, no. 142, 1 October 2016 (2016-10-01), pages 44 - 51, XP055747004
- PILLAI R ET AL: "1,2-Pentanediol - a Multifunctional Ingredient for Personal Care Applications", SOFW JOURNAL, VERLAG FUER CHEMISCHE INDUSTRIE H. ZIOLKOWSKY GMBH, DE, vol. 131, no. 6, 1 June 2005 (2005-06-01), pages 12 - 22, XP002697177, ISSN: 0942-7694

## Description

### Technical Field

This invention relates to the use of a compound for improving the storage stability of a cosmetic.

### Background Art

Antibacterial agents and antiseptic agents are commonly used in cosmetics in order to suppress propagation of bacteria and microorganisms in products. Of these, parabens are used most frequently as antibacterial/antiseptic agents in cosmetics. Parabens exhibit a high antibacterial/antiseptic effect, but cause extremely high skin irritation, and are limited to a usage concentration of 1 mass% or less in cosmetics in Japan. Therefore, the scope of use of parabens can be limited. In addition, the number of people showing allergic reactions to parabens has increased recently, and demand for paraben-free cosmetics is rapidly increasing.

As a result, examples of antibacterial/antiseptic agents able to be blended in cosmetics as paraben replacements include phenoxyethanol, alkane diol compounds such as 1,2-octane diol and glyceryl ether compounds such as 2-ethylhexyl glyceryl ether and n-hexyl glyceryl ether. These compounds exhibit antibacterial performance, cause less skin irritation than parabens, and are highly safe for humans. As a result, these compounds have started to become commonly used in medicinal products and cosmetics in recent years (for example, see Patent Documents 1 to 4).

However, by blending 1,2-octane diol, 2-ethylhexyl glyceryl ether or n-hexyl glyceryl ether in a cosmetic, storage stability may deteriorate and separation or sedimentation may occur, especially in cosmetics having a creamy formulation. Compared to these compounds, however, cases where phenoxyethanol is blended are relatively stable at ordinary temperature (25°C or lower), but in high temperature environments, such as in summer, separation and sedimentation may also occur in the same way as with 1,2-octane diol, 2-ethylhexyl glyceryl ether or n-hexyl glycidyl ether.

Patent Document 5 discloses a cosmetic composition having high antimicrobial property and being superior in preservative quality, which comprises a particular acylproline (component (A)), and a particular hydroxamic acid derivative, or acetophenone derivative (component (B)). It further relates to a method of preserving cosmetics, comprising adding the above components. A diol compound (component (C)) may be used as an optional component.

Patent Document 6 discloses antimicrobial agents such as cyclohexyloxy-1,2-propanediol and their use as preservative in cosmetics. The above compound has an antibacterial effect in a cosmetic.

Patent Document 7 discloses cosmetic compositions exhibiting good antibacterial activity. The composition comprises a compound A which is either one of hexyl glyceryl ether and cyclohexyl glyceryl ether, a compound B which is either one of 1,2-propanediol and 1,3-propanediol, and a component (C). Cyclohexyl glyceryl ether is reported to have antibacterial activity in a cosmetic composition.

Patent Document 8 discloses the use of 3-cyclohexyloxy-1,2-propanediol as antimicrobial agent to preserve cosmetics, optionally in combination with other antibacterial agents, oleyl alcohol and surfactants.

Non-Patent Document 1 discloses properties of the commercial compound ADEKA NOL CHG, which is cyclohexyl glycerol. Bacteriostatic and moisturizing effects and a low level of skin irritation are reported.

Non-Patent Document 2 discloses the multifunctionality of pentylene glycol and in particular the fact that pentylene glycol has good antimicrobial and solubilizing properties. The ability of pentylene glycol to significantly reduce the particle size of cosmetic emulsions, thereby leading to better stability, is also reported.

### Citation List

### Patent Literature

[Patent Document 1] Japanese Patent Application Publication No. H10-265330
[Patent Document 2] Japanese Patent Application Publication No. 2007-145748
[Patent Document 3] Japanese Patent Application Publication No. 2015-086159
[Patent Document 4] Japanese Patent Application Publication No. 2016-029099
[Patent Document 5] EP 3 100 715 A1
[Patent Document 6] JP 2007 291049 A
[Patent Document 7] WO 2015/125392 A1
[Patent Document 8] JP 2011 173808 A

### Non-Patent Literature

[Non-Patent Document 1] "ADEKA NOL CHG Light yellow liquid, Arbitrarily miscible Antibacterial Properties", 9 June 2016
Non-Patent Document 2] "1,2-Pentanediol - a Multifunctional Ingredient for Personal Care Applications", Pillai R. et al., SÖFW, 131(6), 1 June 2005

### Summary of Invention

### Technical Problem

As mentioned above, cosmetics are subject to a variety of constraints, and need to maintain a homogeneous state over a long period of time as products when water-soluble components and oil-soluble components are homogeneously blended. Among compounds able to be used for purposes other than stabilizers, such as antibacterial/antiseptic agents, compounds having a stabilizing effect on cosmetics have been found, and blending and using such compounds in cosmetics is useful from the perspective of reducing the number and amount of additives, such as stabilizers, used.

### Solution to Problem

As a result of diligent research, the inventors of this invention found that compounds represented by general formula (1) below, which exhibit antibacterial/antiseptic properties, contribute to storage stability of cosmetics, and completed this invention. That is, this invention is the use of a compound represented by the following general formula (1) for improving the storage stability of a cosmetic, wherein the cosmetic contains an antibacterial/ antiseptic agent, and wherein the antibacterial/ antiseptic agent is one or more types selected from the group consisting of phenoxyethanol, n-hexyl glyceryl ether, caprylyl glycol and ethylhexylglycerin:. 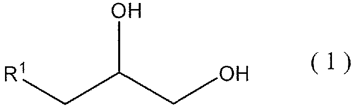 (In the formula, R¹ represents a group represented by general formula (2)) 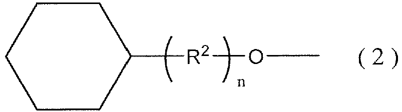 (In the formula, R² represents an alkylene group having 1 to 3 carbon atoms, and n represents an integer of 0 or 1.)

### Advantageous Effects of Invention

In this invention, because it is possible to improve the storage stability of a cosmetic by using a compound represented by general formula (1), which was known in the past to exhibit an antibacterial/antiseptic effect, it can be expected that the number and amount of additives blended such as stabilizers will be reduced. In addition, in cases where this compound is used, it is possible to prevent separation and sedimentation of a cosmetic even in a high temperature high humidity environment, such as in summer. In particular, cosmetics obtained using antibacterial/antiseptic agents such as phenoxyethanol, 1,2-octane diol, 2-ethylhexyl glyceryl ether and n-hexyl glyceryl ether may exhibit inferior stability, and this invention can contribute to stabilizing cosmetics containing any of these compounds.

In addition, because the compound represented by general formula (1) exhibits low skin irritation, it is possible to provide a cosmetic that causes little skin irritation by using this invention.

### Description of Embodiments

This invention is the use of a compound represented by the following general formula (1) for improving the storage stability of a cosmetic as defined above. 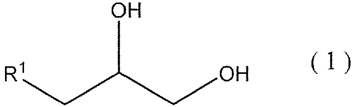 (In the formula, R¹ represents a group represented by general formula (2)) 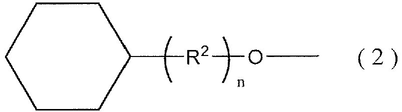 (In the formula, R² represents an alkylene group having 1 to 3 carbon atoms, and n represents an integer of 0 or 1.)

In general formula (1), R¹ represents a group represented by general formula (2).

In general formula (2), R² represents an alkylene group having 1 to 3 carbon atoms, and examples of this group include methylene groups, ethylene groups, propylene groups and isopropylene groups. Of these, methylene groups and ethylene groups are preferred from the perspective of ease of preparation and procurement of raw materials.

Here, n represents an integer of 0 or 1, and it is preferable for n to be 0 from the perspective of being able to obtain a compound that readily achieves the advantageous effect of this invention.

Among such compounds represented by general formula (1), it is preferable to blend a compound in which R¹ in general formula (1) is a group represented by general formula (2) from the perspective of being able to obtain a cosmetic which exhibits an excellent antibacterial/antiseptic effect and causes little skin irritation.

A compound represented by general formula (1) can be obtained by directly producing a compound represented by general formula (1) or by procuring a commercially available product.

The method for producing a compound represented by general formula (1) is not particularly limited, and any publicly known production method can be used.

Of these, cases where R¹ in a compound represented by general formula (1) is a hydrocarbon group having 2 or 3 carbon atoms are preferred from the perspective of simplicity in procuring a commercially available product or producing the compound using Production Method i or Production Method ii below.

### Production Method i

A method for producing a 1,2-diol by reacting hydrogen peroxide with an olefin in the presence of a catalyst.

### Production Method ii

A method for producing a 1,2-diol from an olefin via an epoxide in the presence of an oxidizing agent.

Among Production Methods i and ii and procurement of a commercially available product, procurement of a commercially available product is more preferred from the perspective of simplicity. Examples of commercially available products include products available from Osaka Organic Chemical Industry Ltd., Tokyo Chemical Industry Co., Ltd. and Kokyu Alcohol Kogyo Co., Ltd.

In addition, cases where R¹ in a compound represented by general formula (1) is a group represented by general formula (2) are preferred because producing such a compound using any of Production Methods I to VI below is simple and inexpensive.

### Production Method I

A method comprising subjecting an alcohol compound represented by general formula (3) below and glycerin to a dehydrating condensation reaction. 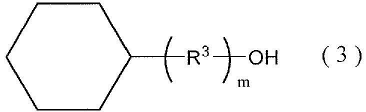 (In the formula, R³ represents an alkylene group having 1 to 3 carbon atoms, and m represents an integer of 0 or 1.)

### Production Method II

A method comprising subjecting an alcohol compound represented by general formula (3) above and 1-chloro-2,3-propane diol to a dehydrochlorination reaction.

### Production Method III

A method comprising reacting an alcohol compound represented by general formula (3) with epichlorohydrin, and then hydrolyzing the thus obtained glycidyl ether compound.

### Production Method IV

A method comprising reacting an alcohol compound represented by general formula (3) with glycidol.

### Production Method V

A method comprising reacting an alcohol compound represented by general formula (3) with allyl chloride or allyl bromide, oxidizing using hydrogen peroxide, and then hydrolyzing the thus obtained glycidyl ether compound.

### Production Method VI

A method comprising subjecting a compound represented by general formula (4) below and glycerin to a dehydrohalogenation reaction. 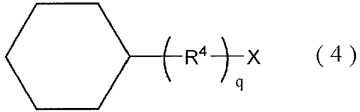 (In the formula, R⁴ represents an alkylene group having 1 to 3 carbon atoms, q represents an integer of 0 or 1, and X represents a halogen atom.)

Of the methods above, Production Method III is more preferred from the perspectives of being simple and inexpensive.

In general formula (3), R³ represents an alkylene group having 1 to 3 carbon atoms. Examples of R³ include a methylene group, an ethylene group, a propylene group and an isopropylene group. Of these, methylene groups and ethylene groups are preferred from the perspective of ease of preparation and procurement of raw materials. m represents an integer of 0 or 1, and it is preferable for m to be 0 from the perspective of being able to obtain a compound that readily achieves the advantageous effect of this invention.

In general formula (4), R⁴ represents an alkylene group having 1 to 3 carbon atoms. Examples of R⁴ include a methylene group, an ethylene group, a propylene group and an isopropylene group. Of these, methylene groups and ethylene groups are preferred from the perspective of ease of preparation and procurement of raw materials. q represents an integer of 0 or 1, and it is preferable for q to be 0 from the perspective of being able to obtain a compound that readily achieves the advantageous effect of this invention.

The amount of the compound represented by general formula (1) used is not particularly limited, but is preferably 0.05 to 5 mass%, more preferably 0.1 to 3 mass%, and further preferably 0.5 to 1 mass%, of the total amount of a cosmetic from the perspective of readily achieving the advantageous effect of this invention.

Compounds represented by general formula (1) exhibit antibacterial/antiseptic properties, cause little skin irritation and are highly safe to humans, and can therefore be used as antibacterial/antiseptic components in the same way as antibacterial/antiseptic agents for cosmetics, and can impart a cosmetic with antibacterial/antiseptic properties when blended in the cosmetic.

Furthermore, in comparison with commonly used antibacterial/antiseptic agents for cosmetics, the compounds represented by general formula (1) exhibit particularly good storage stability when blended in a cosmetic, and can therefore also be used as storage stability-improving agents for cosmetics. Moreover, the storage stability mentioned above is exhibited not only at ordinary temperature (25°C or lower), but also in high temperature environments (40°C to 60°C) that are expected in summer.

Compounds represented by general formula (1) can be used as antibacterial/antiseptic components in combination with commonly used antibacterial/antiseptic agents for cosmetics. In cases where commonly used antibacterial/antiseptic agents for cosmetics are singularly used as antibacterial/antiseptic components, the storage stability of a cosmetic may significantly deteriorate, but by additionally using a compound represented by general formula (1), it is possible to suppress a deterioration in storage stability. Moreover, the storage stability mentioned above is exhibited not only at ordinary temperature (25°C or lower), but also in high temperature environments (40°C to 60°C) that are expected in summer.

The antibacterial/antiseptic agent is one or two types selected from the group consisting of phenoxyethanol, n-hexyl glyceryl ether, caprylyl glycol and ethylhexylglycerin and it is used in combination with the compound represented by general formula (1) from the perspectives of causing little skin irritation, being highly safe for humans and achieving the advantageous effect of this invention to a remarkable degree, and one or more types selected from the group consisting of n-hexyl glyceryl ether and ethylhexylglycerin are particularly preferred.

From the perspective of antibacterial/antiseptic properties, additionally using this type of antibacterial/antiseptic agent for cosmetics is preferred in order to readily obtain a cosmetic which exhibits good storage stability and a high antibacterial/antiseptic effect while suppressing a deterioration in storage stability.

When using a compound represented by general formula (1) together with an antibacterial/antiseptic agent in a cosmetic, the blending proportions of the compound represented by general formula (1) and the antibacterial/antiseptic agent in the cosmetic are not particularly limited as long as the advantageous effect of this invention can be achieved, but from the perspectives of significantly increasing the storage stability of the cosmetic and readily obtaining a cosmetic having a high antibacterial/antiseptic effect, the blending mass ratio is preferably 10:1 to 1:10, more preferably 1:5 to 5:1, and further preferably 1:3 to 3:1.

In cases where a compound represented by general formula (1) is blended in a cosmetic, the blending method is not limited, and the advantageous effect of this invention is sufficiently exhibited whether the compound represented by general formula (1) is blended after being dissolved together with other aqueous components when the cosmetic is produced or is blended after producing the cosmetic in advance from components other than the compound represented by general formula (1).

The type of formulation of a cosmetic referred to in this description is not particularly limited, and examples thereof include liquids, gels, sherbets, milky lotions, creams, ointments, solid pastes, pastes, solids and powders. Of these, it is preferable to obtain a cosmetic having significantly higher storage stability by using this invention in a creamy cosmetic in which storage stability readily deteriorates in cases where a commonly used antibacterial/antiseptic agent for cosmetics is blended. Here, "cream" means a cloudy, viscous formulation that is not fluid.

In addition, examples of creamy cosmetics include oil-free creams that do not contain oil components, water-free creams that do not contain water, oily creams having a high oil content, slightly oily creams having a low oil content and moderately oily creams in which the oil content falls between that of oily creams and slightly oily creams. Of these, cosmetics formulated as oily creams, slightly oily creams and moderately oily creams are preferred from the perspective of being able to exhibit the advantageous effect of this invention to a high degree. In addition, in the case of emulsions obtained by stably dispersing two liquids that do not mix, such as water and oil, such as oily creams, slightly oily creams and moderately oily creams, these emulsions are broadly classified into oil-in-water (O/W) types, in which water is the continuous phase and oily components are dispersed therein, and water-in-oil (W/O) types, in which an oil is the continuous phase and water-soluble components are dispersed therein. However, either type is preferred from the perspective of achieving the advantageous effects of this invention as long as oil components and water components are blended in a well-balanced manner in the cream, and among cosmetic creams in particular, cosmetic creams obtained by blending 20 to 80 mass% of oily components and 80 to 20 mass% of aqueous components are more preferred, and cosmetic creams obtained by blending 20 to 40 mass% of oil components and 80 to 60 mass% of aqueous components are particularly preferred.

A cosmetic used according to this invention preferably contains at least one type selected from the group consisting of surfactants and higher alcohols from the perspective of readily achieving the advantageous effect of this invention and readily maintaining a formulation type such as a liquid, a gel, a sherbet, a milky lotion, a cream, an ointment, a solid paste, a paste, a solid or a powder.

Examples of surfactants include anionic surfactants, cationic surfactants, non-ionic surfactants, and amphoteric surfactants. Examples of non-ionic surfactants include sorbitan fatty acid esters (for example, sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexanoate, and diglycerol sorbitan tetra-2-ethylhexanoate), glyceryl fatty acid esters/polyglyceryl fatty acid esters (for example, glyceryl mono-cottonseed oil fatty acids, glyceryl monoerucate, glyceryl sesquioleate, glyceryl monostearate, glyceryl α,α'-oleate pyroglutamate, glyceryl monostearate malate, polyglyceryl caprylate, polyglyceryl laurate, polyglyceryl myristate, polyglyceryl palmitate, polyglyceryl stearate and polyglyceryl polyricinoleate), propylene glycol fatty acid esters (for example, propylene glycol monostearate), hydrogenated castor oil derivatives, glyceryl alkyl ethers, POE sorbitan fatty acid esters (for example, POE sorbitan monooleate, POE sorbitan monostearate, POE sorbitan monoolate, and POE sorbitan tetraoleate), POE sorbitol fatty acid esters (for example, POE sorbitol monolaurate, POE sorbitol monooleate, POE sorbitol pentaoleate, and POE sorbitol monostearate), POE glyceryl fatty acid esters (for example, POE glyceryl monostearate, POE glyceryl monoisostearate, POE glyceryl triisostearate, and POE monooleate), POE fatty acid esters (for example, POE distearate, POE monostearate, POE mono/dioleate, and ethylene glycol distearate), POE alkyl ethers (for example, POE lauryl ether, POE oleyl ether, POE stearyl ether, POE behenyl ether, POE-2-octyldodecyl ether, and POE cholestanol ether), pluronic surfactants (for example, Pluronic), POE·POP alkyl ethers (for example, POE·POP cetyl ether, POE·POP 2-decyl tetradecyl ether, POE·POP monobutyl ether, POE·POP hydrogenated lanolin, and POE·POP glyceryl ether), tetra-POE·tetra-POP ethylenediamine condensates (for example, Tetronic), POE castor oil/hydrogenated castor oil derivatives (for example, POE castor oil, POE hydrogenated castor oil, POE hydrogenated castor oil monoisostearate, POE hydrogenated castor oil triisostearate, POE hydrogenated castor oil monopyroglutamate monoisostearate diester, and POE hydrogenated castor oil maleate), POE beeswax·lanolin derivatives (for example, POE sorbitol beeswax), alkanolamides (for example, coconut oil fatty acid diethanolamide, lauric acid monoethanolamide, and fatty acid isopropanolamides), POE propylene glycol fatty acid esters, POE alkylamines, POE fatty acid amides, sucrose fatty acid esters, alkylethoxydimethylamine oxides and trioleyl phosphate.

Examples of anionic surfactants include fatty acid soaps (for example, sodium laurate, and sodium palmitate), higher alkyl sulfate ester salts (for example, sodium lauryl sulfate, and potassium lauryl sulfate), alkyl ether sulfate ester salts (for example, POE triethanolamine lauryl sulfate, and POE sodium lauryl sulfate), N-acylsarcosinates (for example, sodium lauroyl sarcosinate), higher fatty acid amide sulfonic acid salts (for example, sodium N-myristyl-N-methyltaurine, sodium coconut oil fatty acid methyltaurine, and sodium laurylmethyltaurine), phosphoric acid ester salts (sodium POE oleyl ether phosphate, and POE stearyl ether phosphate), sulfosuccinic acid salts (for example, sodium di-2-ethylhexyl sulfosuccinate, sodium monolauroyl monoethanolamide polyoxyethylene sulfosuccinate, sodium lauryl and polypropylene glycol sulfosuccinate), alkylbenzene sulfonic acid salts (for example, sodium linear dodecylbenzene sulfonate, triethanolamine n-dodecylbenzene sulfonate, and n-dodecylbenzene sulfonic acid), higher fatty acid ester sulfate ester salts (for example, sodium coconut oil fatty acid glyceryl sulfate), N-acylglutamic acid salts (for example, monosodium N-lauroylglutamate, disodium N-stearoylglutamate, and monosodium N-myristoyl-L-glutamate), sulfated oils (for example, sulfonated castor oil), POE alkyl ether carbonates, POE alkyl allyl ether carboxylic acid salts, α-olefin sulfonic acid salts, higher fatty acid ester sulfonic acid salts, secondary alcohol sulfate ester salts, higher fatty acid alkylolamide sulfate ester salts, sodium lauroyl monoethanolamide succinate, ditriethanolamine N-palmitoyl aspartate and sodium casein.

Examples of cationic surfactants include alkyltrimethyl ammonium salts (for example, stearyltrimethyl ammonium chloride, and lauryltrimethyl ammonium chloride), alkyl pyridinium salts (for example, cetyl pyridinium chloride), distearyldimethyl ammonium chloride dialkyldimethyl ammonium salts, poly(N,N'-dimethyl-3,5-methylenepiperidinium) chloride, alkyl quaternary ammonium salts, alkyldimethylbenzyl ammonium salts, alkyl isoquinolinium salts, dialkyl morpholinium salts, POE alkylamines, alkylamine salts, polyamine fatty acid derivatives, amyl alcohol fatty acid derivatives, benzalkonium chloride and benzethonium chloride, and examples of amphoteric surfactants include imidazoline-based amphoteric surfactants (for example, sodium 2-undecyl-N,N,N-(hydroxyethylcarboxymethyl)-2-imidazoline, and disodium 2-cocoyl-2-imidazolinium hydroxide-1-carboxyethyloxy), betaine type surfactants (for example, 2-heptadecyl-N-carboxymethyl-N-hydroxyethyl imidazolinium betaine, lauryldimethylaminoacetic acid betaine, alkyl betaines, amide betaines, and sulfobetaines).

It is possible to use one or more of the same, or different, types of surfactant mentioned above.

Of these, it is preferable to use a non-ionic surfactant in this invention because a creamy cosmetic is preferred in this invention, and it is more preferable to use one or more surfactants selected from among glyceryl fatty acid esters/polyglyceryl fatty acid esters and POE fatty acid esters.

The amount of the surfactants blended is not particularly limited, but is preferably 0.5 to 30 mass%, more preferably 1 to 15 mass%, and further preferably 2 to 8 mass%, of the total amount of a cosmetic from the perspective of readily achieving the advantageous effect of this invention.

Examples of higher alcohols include primary alcohols having hydrocarbon groups with 8 to 30 carbon atoms (which may be straight chained or branched chain, and saturated or unsaturated). More specifically, examples of higher alcohols include caprylyl alcohol, capryl alcohol, lauryl alcohol, myristyl alcohol, cetyl alcohol, hexyldecanol, stearyl alcohol, isostearyl alcohol, oleyl alcohol, octyl dodecanol, chimyl alcohol, decyltetradecanol, hexyldecanol, arachyl alcohol, behenyl alcohol, carnaubyl alcohol and ceryl alcohol, and it is possible to use one or more of these higher alcohols. Of these, from the viewpoint of obtaining a cream for cosmetics readily exhibiting the advantageous effect of this invention, it is preferable to use one or more types selected from among cetyl alcohol, stearyl alcohol, arachyl alcohol and behenyl alcohol, with behenyl alcohol being more preferred.

The amount of the higher alcohol blended is not particularly limited, but is preferably 0.5 to 30 mass%, more preferably 1 to 15 mass%, and further preferably 2 to 8 mass%, of the total amount of a cosmetic from the perspective of readily achieving the advantageous effect of this invention.

The method of this invention can achieve the advantageous effect of this invention in any cosmetic application, but in order for the advantageous effect of this invention to be achieved to a higher degree, cosmetic applications such as face washes, makeup removers, cold creams for massaging, nourishing creams for foundations, night creams, hand creams, body creams and shaving creams are preferred.

It is possible to blend optional components that are commonly used as cosmetic additives in a cosmetic that uses the method of this invention. Examples of optional components that are commonly used as cosmetic additives include solvents, powder components, oils/fats, waxes, silicone oils, ester oils, hydrocarbon oils, higher fatty acids, moisturizers, water-soluble polymer compounds, metal ion-sequestering agents, sugars, amino acids and derivatives thereof, organic amines, pH-adjusting agents, vitamins, antioxidants, ultraviolet radiation absorbers, fragrances, cosmetic components, blood circulation promoters, antiphlogistic agents, activators, antiseborrheic agents, anti-inflammatory agents and a variety of other extracts, and it is possible to use one or more of these optional components.

Examples of solvents include alcohol compounds such as ethanol, propanol, isopropanol, butanol, propylene glycol, dipropylene glycol, butylene glycol and glycerin.

Examples of powder components include inorganic powders (for example, talc, kaolin, mica, sericite, muscovite, phlogopite, synthetic mica, red mica, biotite, vermiculite, magnesium carbonate, calcium carbonate, aluminum silicate, barium silicate, calcium silicate, magnesium silicate, strontium silicate, metal tungstates, magnesium, silica, zeolites, barium sulfate, calcined calcium sulfate (calcined gypsum), calcium phosphate, fluorapatite, hydroxyapatite, ceramic powders, metal soaps (for example, zinc myristate, calcium palmitate and aluminum stearate), and boron nitride), organic powders (for example, polyamide resin powders (nylon powders), polyethylene powders, polymethyl methacrylate powders, polystyrene powders, styrene-acrylic acid copolymer resin powders, benzoguanamine resin powders, polytetrafluoroethylene powders, and cellulose powders), inorganic white pigments (for example, titanium dioxide, and zinc oxide), inorganic red pigments (for example, iron oxide (red iron oxide), and iron titanate), inorganic brown pigments (for example, γ-iron oxide), inorganic yellow pigments (for example, yellow iron oxide, and loess), inorganic black pigments (for example, black iron oxide, and lower titanium oxides), inorganic violet pigments (for example, manganese violet, and cobalt violet), inorganic green pigments (for example, chromium oxide, chromium hydroxide, and cobalt titanate), inorganic blue pigments (for example, ultramarine blue, and Prussian blue), pearlescent pigments (for example, titanium oxide-coated mica, titanium oxide-coated bismuth oxychloride, titanium oxide-coated talc, colored titanium oxide-coated mica, bismuth oxychloride, and fish scales), metal powder pigments (for example, aluminum powders, and copper powders), organic pigments such as zirconium, barium and aluminum lakes (for example, organic pigments such as Red 201, Red 202, Red 204, Red 205, Red 220, Red 226, Red 228, Red 405, Orange 203, Orange 204, Yellow 205, Yellow 401 and Blue 404; Red 3, Red 104, Red 106, Red 227, Red 230, Red 401, Red 505, Orange 205, Yellow 4, Yellow 5, Yellow 202, Yellow 203, Green 3, and Blue 1), and natural dyes (for example, chlorophyll, and β-carotene).

Examples of oils/fats include avocado oil, camellia oil, turtle oil, macadamia nut oil, corn oil, mink oil, olive oil, rape seed oil, egg yolk oil, sesame oil, persic oil, wheat germ oil, camellia kissi seed oil, castor oil, linseed oil, safflower oil, cottonseed oil, perilla oil, soybean oil, peanut oil, tea seed oil, kaya oil, rice bran oil, Paulownia mikado oil, Paulownia tomentosa oil, jojoba oil, germ oil, triglycerol, cocoa butter, coconut oil, hydrogenated coconut oil, palm oil, palm kernel oil, sumac seed oil, hydrogenated oils, Japan tallow oil, hydrogenated castor oil, rosemary oil, Matricaria chamomilla oil, eucalyptus oil, rice germ oil, wheat germ oil, γ-oryzanol, plant ceramides (glycosylceramides), carrot oil, coix seed extract, Equisetum arvense extract, arnica extract, chamomile extract, lithospermum root extract, Tilia japonica extract, Achillea alpina extract, sage extract, Angelica acutiloba extract, horse chestnut extract, peach leaf extract, rosemary extract, pearl barley extract, loquat extract, borage oil and evening primrose oil.

Examples of waxes include beeswax, candelilla wax, cotton wax, carnauba wax, bayberry wax, insect wax, spermaceti, montan wax, bran wax, lanolin, kapok wax, lanolin acetate, liquid lanolin, sugar cane wax, isopropyl lanolin fatty acids, hexyl laurate, reduced lanolin, jojoba wax, hard lanolin, shellac wax, POE lanolin alcohol ethers, POE lanolin alcohol acetates, POE cholesterol ethers, polyethylene glycol lanolin fatty acids and POE hydrogenated lanolin alcohol ethers.

Examples of silicone oils include chain-like silicone oils such as dimethicone, methyltrimethicone and caprylylmethicone, cyclic silicone oils such as octamethylcyclotetrasiloxane and decamethylcyclopentasiloxane, and phenylsilicone oils such as diphenyldimethicone, bis-phenylpropyldimethicone, phenyltrimethicone, trimethylpentaphenyltrisiloxane, diphenylsiloxyphenyltrimethicone and trimethylsiloxyphenyldimethicone.

Examples of ester oils include isopropyl myristate, octyldodecyl myristate, isopropyl palmitate, hexyldecyl dimethyloctoate, isocetyl stearate, diisobutyl adipate, glyceryl tri-2-ethylhexanoate, trimethylolpropane triisostearate, glyceryl trimyristate, glyceryl trioctanoate, glyceryl triisopalmitate, cetyl 2-ethylhexanoate and 2-ethylhexyl palmitate.

Examples of hydrocarbon oils include liquid paraffin, ozokerite, squalane, pristane, paraffin, ceresin, squalene, Vaseline and microcrystalline waxes.

Examples of higher fatty acids include lauric acid, myristic acid, palmitic acid, stearic acid, behenic acid, oleic acid, undecylenic acid, tall oil fatty acids, isostearic acid, linolic acid, linolenic acid, eicosapentaenoic acid (EPA) and docosahexaenoic acid (DHA).

Examples of moisturizers include polyethylene glycol, erythritol, xylitol, sorbitol, maltitol, chondroitin sulfate, hyaluronic acid, mucoitin sulfate, calonic acid, atelocollagen, cholesteryl-12-hydroxystearate, sodium lactate, bile salts, dl-pyrrolidone carboxylic acid salts, short chain soluble collagen, diglycerol (EO)PO adducts, Rosa roxburghii extract, Achillea millefolium extract and melilot extract.

Examples of water-soluble polymer compounds include starch-based polymers (for example, carboxymethyl starch, and methylhydroxypropyl starch), cellulose-based polymers (methyl cellulose, ethyl cellulose, methylhydroxypropyl cellulose, hydroxyethyl cellulose, sodium cellulose sulfate, hydroxypropyl cellulose, carboxymethyl cellulose, sodium carboxymethyl cellulose, crystalline cellulose, and cellulose powders), alginic acid-based polymers (for example, sodium alginate, and propylene glycol alginate), vinyl-based polymers (for example, polyvinyl alcohol, polyvinyl methyl ether, polyvinylpyrrolidone, and carboxyvinyl polymers), polyoxyethylene-based polymers (for example, polyoxyethylene-polyoxypropylene copolymers prepared from polyethylene glycol 20,000, 40,000 or 60,000), acrylic-based polymers (for example, sodium polyacrylate, polyethyl acrylate, and polyacrylamide), polyethyleneimines and cationic polymers.

Examples of metal ion-sequestering agents include 1-hydroxyethane-1,1-diphosphonic acid, tetrasodium 1-hydroxyethane-1,1-diphosphonate, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid and trisodium ethylenediamine hydroxyethyl tricitrate.

Examples of monosaccharides include trioses (for example, D-glyceryl aldehyde, and dihydroxyacetone), tetroses (for example, D-erythrose, D-erythrulose, and D-threose), pentoses (for example, L-arabinose, D-xylose, L-lyxose, D-arabinose, D-ribose, D-ribulose, D-xylulose, and L-xylulose), hexoses (for example, D-glucose, D-talose, D-psicose, D-galactose, D-fructose, L-galactose, L-mannose, and D-tagatose), heptoses (for example, aldoheptose, and hepulose), octoses (for example, octulose), deoxy sugars (for example, 2-deoxy-D-ribose, 6-deoxy-L-galactose, and 6-deoxy-L-mannose), amino sugars (for example, D-glucosamine, D-galactosamine, sialic acid, aminouronic acid, and muramic acid), uronic acids (for example, D-glucuronic acid, D-mannuronic acid, L-guluronic acid, D-galacturonic acid, and L-iduronic acid).

Examples of oligosaccharides include sucrose, umbelliferose, lactose, planteose, isolychnose, α,α-trehalose, raffinose, lychnose, umbilicin, stachyose and verbascose.

Examples of polysaccharides include cellulose, queen's seed, chondroitin sulfate, starch, galactan, dermatan sulfate, glycogen, gum Arabic, heparan sulfate, hyaluronic acid, gum tragacanth, keratan sulfate, chondroitin, xanthan gum, mucoitin sulfate, guar gum, dextran, keratosulfate, locust bean gum, succinoglycan and calonic acid.

Examples of amino acids include neutral amino acids (for example, threonine, and cysteine) and basic amino acids (for example, hydroxylycine). In addition, examples of amino acid derivatives include sodium acyl sarcosinate (sodium lauroyl sarcosinate), acyl glutamates, sodium acyl β-alanine, glutathione and pyrrolidone carboxylic acid.

Examples of organic amines include monoethanolamine, diethanolamine, triethanolamine, morpholine, triisopropanolamine, 2-amino-2-methyl-1,3-propane diol and 2-amino-2-methyl-1-propanol.

Examples of pH-adjusting agents include buffering agents such as lactic acid-sodium lactate, citric acid-sodium citrate and succinic acid-sodium succinate.

Examples of vitamins include vitamin E and derivatives thereof, vitamin C and derivatives thereof, vitamin D and derivatives thereof, vitamin F and derivatives thereof, vitamin K and derivatives thereof, vitamin A and derivatives thereof, and vitamin B derivatives, but are not limited to these. Specific examples include γ-tocopherol, stearyl ascorbate, ascorbyl dipalmitate, tocopherol nicotinate, menadione, dehydrocholesterol, ergocalciferol, pyridoxine dicaprylate, ascorbyl tetra-hexyldecanoate (VCIP), retinol, retinol derivatives such as retinol palmitate and retinol acetate, docosahexaenoic acid, linolic acid, pantenol, tocopherol linolenate, isopropyl linolate, linolenic acid, pyridoxine palmitate, vitamin A, β-carotene, pyridoxine dipalmitate, phylloquinone, pantothenic acid and derivatives thereof, and biotin.

Examples of antioxidants include dibutylhydroxytoluene, butylhydroxyanisole, sorbic acid, sodium sulfite, sodium hydrogen sulfite, sodium thiosulfate, metabisulfites, thiotaurine, hypotaurine, thioglycerols, thiourea, thioglycolic acid, cysteine hydrochloride, propyl gallate, gallic acid derivatives, ascorbic acid, ascorbic acid derivatives (ascorbic acid phosphate esters), tocopherols, tocopherol derivatives, erythorbic acid, p-t-butylphenol, phytic acid and L-cysteine hydrochloride.

Examples of ultraviolet radiation absorbers include benzoic acid-based ultraviolet radiation absorbers, anthranilic acid-based ultraviolet radiation absorbers, salicylic acid-based ultraviolet radiation absorbers, cinnamic acid-based ultraviolet radiation absorbers, benzophenone-based ultraviolet radiation absorbers, benzotriazole-based ultraviolet radiation absorbers, triazine-based ultraviolet radiation absorbers, benzoate-based ultraviolet radiation absorbers, cyanoacrylate-based ultraviolet radiation absorbers, oxanilide-based ultraviolet radiation absorbers and formamidine-based ultraviolet radiation absorbers. Examples of benzoic acid-based ultraviolet radiation absorbers include para-aminobenzoic acid, ethyl para-aminobenzoate, ethylhexyl para-dimethylaminobenzoate, octyl para-dimethylaminobenzoate, amyl para-dimethylaminobenzoate, monoglyceryl para-aminobenzoate, glyceryl para-aminobenzoate, ethyldihydroxypropyl glyceryl para-aminobenzoate, ethyl N,N-dipropoxypara-aminobenzoate, ethyl N,N-diethoxypara-aminobenzoate, ethyl N,N-dimethylpara-aminobenzoate, butyl N,N-dimethylpara-aminobenzoate, amyl N,N-dimethylpara-aminobenzoate, octyl N,N-dimethylpara-aminobenzoate and hexyl diethylaminohydroxybenzoylbenzoate. Examples of anthranilic acid-based ultraviolet radiation absorbers include homomenthyl-N-acetyl anthranilate.

Examples of salicylic acid-based ultraviolet radiation absorbers include salicylic acid and sodium salts thereof, amyl salicylate, menthyl salicylate, homomenthyl salicylate, octyl salicylate, phenyl salicylate, benzyl salicylate and p-isopropanolphenyl salicylate. Examples of cinnamic acid-based ultraviolet radiation absorbers include octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, 2-ethylhexyl p-methoxycinnamate (2-ethylhexyl para-methoxycinnamate), 2-ethoxyethyl-p-methoxycinnamate (cinoxate), cyclohexyl-p-methoxycinnamate, ethyl-α-cyano-β-phenyl cinnamate, 2-ethylhexyl α-cyano-β-phenyl cinnamate (octocrylene), glyceryl mono-2-ethylhexanoyl-di-paramethoxy cinnamate, ferulic acid and derivatives thereof.

Examples of benzophenone-based ultraviolet radiation absorbers include 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone (oxybenzone-3), 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone and 5,5'-methylenebis(2-hydroxy-4-methoxybenzophenone).

Examples of benzotriazole-based ultraviolet radiation absorbers include 2-(2-hydroxy-5-methylphenyl)benzotriazole, 2-(2-hydroxy-5-methylphenyl)-5-chlorobenzotriazole, 2-(2-hydroxy-5-tert-octylphenyl)benzotriazole, 2-(2-hydroxy-5-tert-octylphenyl)-5-chlorobenzotriazole, 2-(2-hydroxy-3,5-di-tert-butylphenyl)benzotriazole, 2-(2-hydroxy-3,5-di-tert-butylphenyl)-5-chlorobenzotriazole, 2-(2-hydroxy-3-tert-butyl-5-methylphenyl)benzotriazole, 2-(2-hydroxy-3-tert-butyl-5-methylphenyl)-5-chlorobenzotriazole, 2-(2-hydroxy-3,5-dicumylphenyl)benzotriazole, 2-(2-hydroxy-3,5-dicumylphenyl)-5-chlorobenzotriazole, 2,2'-methylenebis(4-tert-octyl-6-benzotriazolylphenol), polyethylene glycol esters of 2-(2-hydroxy-3-tert-butyl-5-carboxyphenyl)benzotriazole, 2-[2-hydroxy-3-(2-acryloyloxyethyl)-5-methylphenyl]benzotriazole, 2-[2-hydroxy-3-(2-acryloyloxyethyl)-5-methylphenyl]-5-chlorobenzotriazole, 2-[2-hydroxy-3-(2-methacryloyloxyethyl)-5-tert-butylphenyl]benzotriazole, 2-[2-hydroxy-3-(2-methacryloyloxyethyl)-5-tert-butylphenyl]-5-chlorobenzotriazole, 2-[2-hydroxy-3-(2-methacryloyloxyethyl)-5-tert-octylphenyl]benzotriazole, 2-[2-hydroxy-3-(2-methacryloyloxyethyl)-5-tert-octylphenyl]-5-chlorobenzotriazole, 2-[2-hydroxy-3-(2-methacryloyloxyethyl)-5-tert-butylphenyl]benzotriazole, 2-[2-hydroxy-3-(2-methacryloyloxyethyl)-5-tert-butylphenyl]-5-chlorobenzotriazole, 2-[2-hydroxy-5-(2-methacryloyloxyethyl)phenyl]benzotriazole, 2-[2-hydroxy-5-(2-methacryloyloxyethyl)phenyl]-5-chlorobenzotriazole, 2-[2-hydroxy-3-tert-butyl-5-(2-methacryloyloxyethyl)phenyl]benzotriazole, 2-[2-hydroxy-3-tert-butyl-5-(2-methacryloyloxyethyl)phenyl]-5-chlorobenzotriazole, 2-[2-hydroxy-3-tert-amyl-5-(2-methacryloyloxyethyl)phenyl]benzotriazole, 2-[2-hydroxy-3-tert-amyl-5-(2-methacryloyloxyethyl)phenyl]-5-chlorobenzotriazole, 2-[2-hydroxy-3-tert-butyl-5-(3-methacryloyloxypropyl)phenyl]benzotriazole, 2-[2-hydroxy-3-tert-butyl-5-(3-methacryloyloxypropyl)phenyl]-5-chlorobenzotriazole, 2-[2-hydroxy-4-(2-methacryloyloxymethyl)phenyl]benzotriazole, 2-[2-hydroxy-4-(2-methacryloyloxymethyl)phenyl]-5-chlorobenzotriazole, 2-[2-hydroxy-4-(3-methacryloyloxy-2-hydroxypropyl)phenyl]benzotriazole, 2-[2-hydroxy-4-(3-methacryloyloxy-2-hydroxypropyl)phenyl]-5-chlorobenzotriazole, 2-[2-hydroxy-4-(3-methacryloyloxypropyl)phenyl]benzotriazole and 2-[2-hydroxy-4-(3-methacryloyloxypropyl)phenyl]-5-chlorobenzotriazole.

Examples of triazine-based ultraviolet radiation absorbers include 2,4,6-tris[4-(2-ethylhexyloxycarbonyl)anilino]-1,3,5-triazine, 2,4-bis-[{4-(2-ethylhexyloxy)-2-hydroxy}-phenyl]-6-(4-methoxyphenyl)-1,3,5-triazine, 2-(2-hydroxy-4-methoxyphenyl)-4,6-diphenyl-1,3,5-triazine, 2-(2-hydroxy-4-hexyloxyphenyl)-4,6-diphenyl-1,3,5-triazine, 2-(2-hydroxy-4-octoxyphenyl)-4,6-bis(2,4-dimethylphenyl)-1,3,5-triazine, 2-[2-hydroxy-4-(3-C₁₂-C₁₃ mixed alkoxy-2-hydroxypropoxy)phenyl]-4,6-bis(2,4-dimethylphenyl)-1,3,5-triazine, 2-[2-hydroxy-4-(2-acryloyloxyethoxy)phenyl]-4,6-bis(4-methylphenyl)-1,3,5-triazine, 2-[2-hydroxy-4-(2-acetyloxyethoxy)phenyl]-4,6-bisphenyl-1,3,5-triazine, 2-(2,4-dihydroxy-3-allylphenyl)-4,6-bis(2,4-dimethylphenyl)-1,3,5-triazine and 2,4,6-tris(2-hydroxy-3-methyl-4-hexyloxyphenyl)-1,3,5-triazine. Examples of benzoate-based ultraviolet radiation absorbers include resorcinol monobenzoate, 2,4-di-tert-butylphenyl-3,5-di-tert-butyl-4-hydroxybenzoate, octyl (3,5-di-tert-butyl-4-hydroxy)benzoate, dodecyl (3,5-di-tert-butyl-4-hydroxy)benzoate, tetradecyl (3,5-di-tert-butyl-4-hydroxy) benzoate, hexadecyl (3,5-di-tert-butyl-4-hydroxy) benzoate, octadecyl (3,5-di-tert-butyl-4-hydroxy) benzoate, behenyl (3,5-di-tert-butyl-4-hydroxy)benzoate and stearyl (3,5-di-tert-butyl-4-hydroxybenzoate.

Examples of cyanoacrylate-based ultraviolet radiation absorbers include ethyl-α-cyano-β,β-diphenyl acrylate and methyl-2-cyano-3-methyl-3-(p-methoxyphenyl) acrylate. Examples of oxanilide-based ultraviolet radiation absorbers include 2-ethyl-2'-ethoxyoxanilide and 2-ethoxy-4'-dodecyloxanilide. Examples of formamidine-based ultraviolet radiation absorbers include N,N'-diphenyl-N'-(4-ethoxycarbonylphenyl)formamidine, N'-(4-ethoxycarbonylphenyl)-N-methyl-N-phenylformamidine, N,N'-bis(4-ethoxycarbonylphenyl)-N-methylformamidine, N'-(4-ethoxycarbonylphenyl)-N-(2'-methoxyphenyl)-N-methylformamidine and N-(4-n-butoxycarbonylphenyl)-N'-(4'-ethylcarbonyl)-N-methylformamidine.

Examples of other ultraviolet radiation absorbers include 3-(4'-methylbenzylidene)-d,1-camphor, 3-benzylidene-d,l-camphor, 2-phenyl-5-methylbenzoxazole, dibenzalazine, dianisoylmethane, 5-(3,3-dimethyl-2-norbornilidene)-3-pentan-2-one, 4-t-butylmethoxydibenzoylmethane, octyl triazone, urocanic acid, ethyl urocanate, 1-(3,4-dimethoxyphenyl)-4,4-dimethyl-1,3-pentane dione, 2-ethylhexyl dimethoxybenzylidene dioxoimidazolidine propionate, phenylbenzimidazole sulfonic acid, terephthalylidine dicamphor sulfonic acid, drometrizole trisiloxane, methyl anthranilate, rutin, rutin derivatives, oryzanol and oryzanol derivatives.

Examples of fragrances include compound fragrances containing natural fragrances and/or synthetic fragrances. Specifically, examples of natural fragrances include amyris oil, ambrette seed oil, ylang ylang oil, ylang ylang absolute, iris resinoid, iris absolute, iris oil, wintergreen oil, estragon oil, elemi oleoresin, elemi resinoid absolute, elemi tincture, oakmoss concrete, oakmoss absolute, oakmoss resin, oakmoss resinoid, devilwood absolute, devilwood concrete, opopanax resinoid, opopanax absolute, opopanax oil, frankincense resinoid, frankincense absolute, frankincense oil, all spice oil, origanum oil, oregano oil, oregano oleoresin, orange flower absolute, orange flower concrete, kananga oil, gurjun balsam, gurjun balsam oil, cassie absolute, cassie flower oil, cassia oil, cape jasmine absolute, carnation absolute, cabreuva oil, chamomile oil, cardamom oil, galbanum oil, galbanum resin, galbanum resinoid, caraway seed oil, carrot seed oil, litsea cubeba oil, guaicum wood oil, guaicum resin, guaicum concrete, cinnamomum camphora oil, cumin oil, cumin absolute, cumin oleoresin, clary sage oil, grapefruit oil, clove oil, costus oil, copaiba balsam, copaiba balsam oil, copaiba balsam resin, coriander oil, sandalwood oil, perilla oil, cedarwood oil, citronella oil, jasmine oil, jasmine absolute, jasmine concrete, juniper berry oil, genet absolute, jonquil absolute, ginger oil, cinnamon oil, cinnamon bark oil, cinnamon leaf oil, Japanese cedar oil, star anise oil, styrax oil, styrax resinoid, spike lavender oil, spearmint oil, savory oil, sage oil, cedar oil, cedar leaf oil, geranium oil, celery seed oil, thyme oil, taguette oil, tangerine oil, tuberose absolute, tea tree oil, tree moss absolute, tonka bean oil, true balsam, nutmeg oil, narcissus absolute, neroli oil, violet leaf absolute, pine oil, pine needle oil, basil oil, parsley leaf oil, parsley seed oil, parsley herb oil, patchouli oil, peppermint oil, vanilla absolute, honeysuckle absolute, palmarosa oil, valerian oil, bitter orange oil, hyssop oil, Japanese cypress oil, white cedar oil, hyacinth absolute, fennel oil, fig absolute, petitgrain oil, buchu oil, bay oil, vetiver oil, pepper oil, peppermint absolute, peppermint oil, bergamot oil, Peru balsam, benzoin tincture, benzoin resinoid, Cinnamomum camphora oil, marjoram oil, mandarin oil, satsuma oil, mimosa concrete, mimosa absolute, mimosa oil, stag seaweed resinoid, stag seaweed absolute, stag seaweed oil, musk absolute, musk tincture, eucalyptus oil, yuzu oil, lime oil, labdanum oil, labdanum resinoid, lavender oil, lavender absolute, Lavandula burnatii oil, Lavandula burnatii absolute, lemon oil, lemongrass oil, rose oil, rose absolute, rose concrete, rosemary oil, laurel oil and laurel leaf oil.

In addition, examples of synthetic fragrances include ambrettolide, C₆-C₁₂ aldehydes, anisic aldehyde, acetal R, acetophenone, acetyl cedrene, adoxal, allyl amyl glycolate, allyl cyclohexanepropionate, ambroxan, amylcinnamicaldehyde, amylcinnamicaldehyde dimethyl acetal, amyl valerianate, amyl salicylate, acetyl eugenol, isoamyl acetate, isoamyl salicylate, indole, ionone, isobornyl acetate, isocyclocitral, Iso E Super, isoeugenol, isononyl acetate, isobutylquinoline, γ-undecalactone, ethylene brassylate, ethylene dodecanedioate, ethylvanillin, 2-ethylhexanol, aurantiol, 10-oxahexadecanolide, 11-oxahexadecanolide, 12-oxahexadecanolide, oxahexadecen-2-one, eugenol, orivone, oxyphenylone, galaxolide, caryophyllene, cashmeran, carvone, β-caryophyllene, Calone, coumarin, p-cresyl methyl ether, geraniol, geranyl acetate, geranyl formate, geranyl nitrile, Koavone, Sandalore, Sandela, Santalex, cinnamic alcohol, cinnamaldehyde, cis-jasmon, citral, citral dimethyl acetal, citrasal, citronellal, citronellol, citronellyl acetate, citronellyl formate, citronellyl nitrile, cyclaset, cyclamen aldehyde, cyclaprop, dimethyl benzyl carbinol, dihydrojasmone, dihydrolinalool, dihydromyrcenol, Dimetol, dimyrcetol, diphenyl oxide, jasmal, jasmolactone, jasmophyllan, cinnamyl acetate, cyclopentadecanone, cyclohexadecanone, cyclopentadecanolide, cyclohexadecanolide, dimethyl benzyl carbinyl acetate, jasmacyclene, styralyl acetate, styralyl propionate, cedramber, cedryl acetate, cedrol, selestride, α-damascone, β-damascone, δ-damascone, damascenones, terpineol, terpinyl acetate, thymol, tetrahydrolinalool, tetrahydrolinalyl acetate, tetrahydrogeraniol, tetrahydrogeranyl acetate, tonalide, traseolide, Triplal, neryl acetate, nerol, neobergamate, γ-nonalactone nopyl alcohol, nopyl acetate, Bacdanol, hydrotropic alcohol, α-pinene, β-pinene, hydroxycitronellal, hyacinth dimethyl acetal, butyl butyrate, p-t-butylcyclohexanol, p-t-butylcyclohexyl acetate, o-t-butylcyclohexanol, o-t-butylcyclohexyl acetate, fruitate, phentyl alcohol, phenyl ethyl phenyl acetate, phenyl ethyl acetate, pentalide, verdox, benzyl acetate, benzyl alcohol, benzyl salicylate, bergamyl acetate, benzaldehyde, benzyl formate, hedione, helional, heliotropine, cis-3-hexenol, cis-3-hexenyl acetate, cis-3-hexenyl salicylate, hexylcinnamicaldehyde, hexyl salicylate, bornyl acetate, borneol, manzanate, Mayol, myrcene, myrac aldehyde, muguet aldehyde, mugol, musk TM-11, musk 781, musk C₁₄, muscone, musk ketone, musk tibetine, menthanyl acetate, menthonate, methyl anthranilate, methyl eugenol, menthol, α-methylionone, β-methylionone, γ-methylionone, methyl isoeugenol, methyl lavender ketone, methyl salicylate, 14-methyl-hexadecenolide, 14-methyl-hexadecanolide, methyl naphthyl ketone, methyl phenyl acetate, yara yara, δ-C₆-C₁₃ lactones, lime oxide, γ-C₆-C₁₃ lactones, raspberry ketone, limonene, ligustral, lilial, linalool, linalool oxide, linalyl acetate, lyral, rhubafuran, rosephenone, rose oxide and vanillin.

Moreover, the natural fragrances and/or synthetic fragrances listed above can be dissolved as flavor bases in a variety of solvents and blended as water-soluble fragrances or oil-soluble fragrances.

Examples of cosmetic components include placenta extract liquids, mulberry bark extracts, meadow saxifrage extracts, perilla extracts, white mustard extracts and hydrolyzates thereof, white mustard fermentation products, damask rose extracts, Chinese peony extracts and hydrolyzates thereof, lactobacillus fermented rice, lotus seed extracts and hydrolyzates thereof, lotus seed fermentation products, Codonopsis pilosul extracts, pearl barley hydrolyzates, pearl barley fermentation products, royal jelly fermentation products, sake lees fermentation products, Pandanus Amaryllifolius extracts, Arcangelicia flava extracts, kiwi extracts, Matricaria chamomilla extracts, Common Glasswort extracts, Oryza sativa leaf extracts and hydrolyzates thereof, eggplant (water eggplant, long eggplant, kamo eggplant, rice eggplant) extracts and hydrolyzates thereof, extracts of seaweed such as E. gelatinae J. Ag., extracts of marine phonerogram plants such as eelgrass, soy milk fermentation products, jellyfish water, rice fermentation extracts, linolic acid and derivatives and processed products thereof (for example, liposomal linolic acid), animal-derived and fish-derived collagen and derivatives thereof, elastin and derivatives thereof, glycyrrhizinic acid and derivatives (dicalcium salt) thereof, t-cycloamino acid derivatives, allantoin, arbutin, diisopropylamine dichloroacetate, γ-amino-β-hydroxybutyric acid, Gentiana extracts, Glycyrrhiza uralensis extracts, carrot extracts, aloe extracts, Laminaria angastata extracts, Ulva pertusa extracts, Rhamnoceae Zizyphus joazeiro extracts and immature peach extracts.

Examples of other components able to be blended include blood circulation promoters (for example, benzyl nicotinate, β-butoxyethyl nicotinate, capsaicin, zingerone, cantharidis tincture, ichthammol, tannic acid, α-borneol, tocopherol nicotinate, inositol hexanicotinate, cyclandelate, cinnarizine, tolazoline, acetylcholine, verapamil, cepharanthine, and γ-oryzanol), antiphlogistic agents (for example, glycyrrhizinic acid derivatives, glycyrrhetinic acid derivatives, salicylic acid derivatives, hinokitiol, zinc oxide, and allantoin), activators (for example, royal jelly, photosensitizers, and cholesterol derivatives), antiseborrheic agents (for example, sulfur, and thianthol), anti-inflammatory agents (for example, tranexamic acid, thiotaurine, and hypotaurine), and other extracts (for example, Phellodendron amurense, Coptis japonica, Lithospermum erythrorhizon, Chinese peony, Japanese green gentian, birch, sage, Eriobotrya japonica, carrot, aloe, common mallow, iris, grape, coix seed, sponge cucumber, lily, saffron, Cnidium officinale, ginger, Hypericum erectum, Restharrows, garlic, red pepper, Citrus reticulata peel, Angelica acutiloba, and seaweed).

### Examples

This invention will now be explained in detail through the use of examples. However, this invention is in no way limited to these examples, and may be altered as long as such changes to not deviate from the scope of this invention. Moreover, in the examples etc. given below, % means mass percentage unless explicitly indicated otherwise.

### Creamy cosmetics (I)

Compounds represented by general formula (1) and having antibacterial/antiseptic properties, which are used in the examples, are as follows.

### <Compounds represented by general formula (1)>

Compound (1)-1: Pentylene glycol (a compound in which R¹ in general formula (1) is an ethyl group). Compositions comprising a compound (1) -1 are not according to the use of the present invention.
Compound (1)-2: Cyclohexyl glyceryl ether (a compound in which R¹ in general formula (1) is a group represented by general formula (2) and n is 0)

Antibacterial/antiseptic agents commonly blended in cosmetics, which are used in the comparative examples, are as follows.

### <Antibacterial/antiseptic agents>

Phenoxyethanol
n-hexyl glyceryl ether
Caprylyl glycol
Ethylhexylglycerin

### [Cosmetic storage stability test]

Components of the creamy cosmetics (I) used in the examples and comparative examples are shown in Table 1.

**Table 1**

| Component | Amount (mass%) |
|---|---|
| PEG-40 stearate | 2.50 |
| Glyceryl monostearate | 2.00 |
| Behenyl alcohol | 3.00 |
| Dipentaerythrityl hexa(hydroxystearic acid / stearic acid / rosin acid) | 5.00 |
| Liquid paraffin | 20.00 |
| 1,3-butylene glycol | 10.00 |
| Glycerin | 2.00 |
| Xanthan gum | 0.10 |
| Purified water | Balance |
| Antibacterial/antiseptic component | (X) |
| Total | 100.00 |

In the creamy cosmetics (I) shown in Table 1, PEG-40 stearate and glyceryl monostearate were used as surfactants, behenyl alcohol (a higher alcohol), dipentaerythrityl hexa(hydroxystearic acid / stearic acid / rosin acid) and liquid paraffin were used as oil phase components, and 1,3-butylene glycol, glycerin, xanthan gum and water were used as aqueous phase components.

Oil-in-water type creamy cosmetics (I) were obtained by producing cosmetics using components other than antibacterial/antiseptic components, and then adding antibacterial/antiseptic components. More specifically, the creamy cosmetics (I)-1 to (I)-20 below were prepared by heating and dissolving surfactants and oil phase components, separately heating and dissolving aqueous phase components in another system, adding the aqueous phase components to the oil phase components, obtaining a cosmetic by means of phase inversion emulsification, and then adding a compound represented by general formula (1) and/or antibacterial/antiseptic agent listed above (see Table 2). Moreover, creamy cosmetics (I)-13 to (I)-20, which were obtained using both a compound represented by general formula (1) and an antibacterial/antiseptic agent, used 0.5 mass% of each of the compound represented by general formula (1) and the antibacterial/antiseptic agent, that is, a total 1.0 mass%.

**Table 2**

| Cosmetic | Antibacterial/ant iseptic component (X) | Antibacterial/antiseptic agent | Amount (mass%) of blended Antibacterial/antiseptic component (X) and/or Antibacterial/antiseptic component |
|---|---|---|---|
| Creamy cosmetic (I)-1 | Pentylene glycol | - | 0.50 |
| Creamy cosmetic (I)-2 | Pentylene glycol | - | 1.00 |
| Creamy cosmetic (I)-3 | Cyclohexyl glyceryl ether | - | 0.50 |
| Creamy cosmetic (I)-4 | Cyclohexyl glyceryl ether | - | 1.00 |
| Creamy cosmetic (I)-5 | - | Phenoxyethanol | 0.50 |
| Creamy cosmetic (I)-6 | - | Phenoxyethanol | 1.00 |
| Creamy cosmetic (I)-7 | - | n-hexyl glyceryl ether | 0.50 |
| Creamy cosmetic (I)-8 | - | n-hexyl glyceryl ether | 1.00 |
| Creamy cosmetic (I)-9 | - | Caprylyl glycol | 0.50 |
| Creamy cosmetic (I)-10 | - | Caprylyl glycol | 1.00 |
| Creamy cosmetic (I)-11 | - | Ethylhexylglycerin | 0.50 |
| Creamy cosmetic (I)-12 | - | Ethylhexylglycerin | 1.00 |
| Creamy cosmetic (I)-13 | Pentylene glycol | Phenoxyethanol | Total 1.00 (0.50+0.50) |
| Creamy cosmetic (I)-14 | Cyclohexyl glyceryl ether | Phenoxyethanol | Total 1.00 (0.50+0.50) |
| Creamy cosmetic (I)-15 | Pentylene glycol | n-hexyl glyceryl ether | Total 1.00 (0.50+0.50) |
| Creamy cosmetic (I)-16 | Cyclohexyl glyceryl ether | n-hexyl glyceryl ether | Total 1.00 (0.50+0.50) |
| Creamy cosmetic (1)-17 | Pentylene glycol | Caprylyl glycol | Total 1.00 (0.50+0.50) |
| Creamy cosmetic (1)-18 | Cyclohexyl glyceryl ether | Caprylyl glycol | Total 1.00 (0.50+0.50) |
| Creamy cosmetic (1)-19 | Pentylene glycol | Ethylhexylglycerin | Total 1.00 (0.50+0.50) |
| Creamy cosmetic (I)-20 | Cyclohexyl glyceryl ether | Ethylhexylglycerin | Total 1.00 (0.50+0.50) |

Next, the creamy cosmetics described above were subjected to storage stability tests. Each of the obtained creamy cosmetics (I)-1 to (I)-20 was placed in a polyethylene container having a diameter of 4 cm and a height of 6 cm and then subjected to a storage stability test at 50°C for one month. The evaluation method involved visual confirmation according to the criteria below. The results are shown in Table 3.

### [Evaluation method]

A smooth creamy state was maintained with no separation or sedimentation: A
Less than 5 mm of separation or sedimentation of aqueous components was observed: B
At least 5 mm but less than 1 cm of separation or sedimentation of aqueous components was observed: C
At least 1 cm of separation or sedimentation of aqueous components was observed: D

**Table 3**

| | Cosmetic used | Storage stability test evaluation result |
|---|---|---|
| Example 1 | Creamy cosmetic (I)-1 | A |
| Example 2 | Creamy cosmetic (I)-2 | A |
| Example 3 | Creamy cosmetic (I)-3 | A |
| Example 4 | Creamy cosmetic (I)-4 | A |
| Comparative Example 1 | Creamy cosmetic (I)-5 | B |
| Comparative Example 2 | Creamy cosmetic (I)-6 | C |
| Comparative Example 3 | Creamy cosmetic (I)-7 | C |
| Comparative Example 4 | Creamy cosmetic (I)-8 | D |
| Comparative Example 5 | Creamy cosmetic (I)-9 | C |
| Comparative Example 6 | Creamy cosmetic (I)-10 | D |
| Comparative Example 7 | Creamy cosmetic (I)-11 | C |
| Comparative Example 8 | Creamy cosmetic (I)-12 | D |
| Example 5 | Creamy cosmetic (I)-13 | A |
| Example 6 | Creamy cosmetic (I)-14 | A |
| Example 7 | Creamy cosmetic (I)-15 | A |
| Example 8 | Creamy cosmetic (I)-16 | A |
| Example 9 | Creamy cosmetic (I)-17 | A |
| Example 10 | Creamy cosmetic (I)-18 | A |
| Example 11 | Creamy cosmetic (I)-19 | A |
| Example 12 | Creamy cosmetic (I)-20 | A |

The results show that Examples 1 to 4, which used the method of this invention, exhibited extremely good storage stability in high temperature environments that are expected in summer. In addition, it was observed that creamy cosmetics that exhibited poor storage stability in Comparative Examples 1, 3, 5 and 7 showed a significant improvement in storage stability when a compound represented by general formula (1) was additionally used (Examples 5 to 12). That is, it is possible to obtain a cosmetic that exhibits not only the antibacterial/antiseptic effect of a compound represented by general formula (1), but also the antibacterial/antiseptic effect inherent in phenoxyethanol, n-hexyl glyceryl ether, caprylyl glycol or ethylhexylglycerin, and exhibits good storage stability.

### Creamy cosmetics (II)

Compounds represented by general formula (1) and having antibacterial/antiseptic properties, which are used in the examples, are as follows.

### <Compounds represented by general formula (1)>

Compound (1)-1: Pentylene glycol (a compound in which R¹ in general formula (1) is an ethyl group). Compositions comprising a compound (1)-1 are not according to the use of the present invention.
Compound (1)-2: Cyclohexyl glyceryl ether (a compound in which R¹ in general formula (1) is a group represented by general formula (2) and n is 0)

Antibacterial/antiseptic agents used in the comparative examples are as follows.

### <Antibacterial/antiseptic agents>

Phenoxyethanol
n-hexyl glyceryl ether
Caprylyl glycol
Ethylhexylglycerin

### [Cosmetic storage stability test]

Components of the creamy cosmetics (II) used in the examples and comparative examples are shown in Table 4.

**Table 4**

| Component | Amount (mass%) |
|---|---|
| Polyglyceryl-6 ricinoleate | 1.50 |
| Polyglyceryl-10 stearate | 0.50 |
| Squalane | 8.00 |
| Vaseline | 4.00 |
| Triethylhexanoin | 4.00 |
| Diphenylsiloxyphenyltrimethicone | 5.00 |
| Glycerin | 5.00 |
| Sorbitol | 10.00 |
| Common salt | 2.00 |
| Purified water | Balance |
| Antibacterial/antiseptic component | (Y) |
| Total | 100.00 |

In the creamy cosmetics (II) shown in Table 4, polyglyceryl-6 ricinoleate and polyglyceryl-10 stearate were used as surfactants, squalane, Vaseline, triethylhexanoin and diphenylsiloxyphenyltrimethicone were used as oil phase components, and glycerin, sorbitol, common salt and water were used as aqueous phase components.

Water-in-oil type creamy cosmetics (II) were obtained by producing cosmetics using components other than antibacterial/antiseptic components, and then adding antibacterial/antiseptic components. More specifically, the creamy cosmetics (II)-1 to (II)-8 below were prepared by heating and dissolving surfactants and oil phase components, separately heating and dissolving aqueous phase components in another system, adding the aqueous phase components to the oil phase components, obtaining a cosmetic by means of phase inversion emulsification, and then adding a compound represented by general formula (1) and/or antibacterial/antiseptic agent listed above (see Table 5).

**Table 5**

| Cosmetic | Antibacterial/antiseptic component (Y) | Antibacterial/antiseptic agent | Amount (mass%) of blended Antibacterial/antiseptic component (Y) or Antibacterial/antiseptic component |
|---|---|---|---|
| Creamy cosmetic (II)-1 | Pentylene glycol | - | 0.25 |
| Creamy cosmetic (II) -2 | Pentylene glycol | - | 0.50 |
| Creamy cosmetic (II) -3 | Cyclohexyl glyceryl ether | - | 0.25 |
| Creamy cosmetic (II) -4 | Cyclohexyl glyceryl ether | - | 0.50 |
| Creamy cosmetic (II) -5 | - | Ethylhexylglycerin | 0.25 |
| Creamy cosmetic (II)-6 | - | Caprylyl glycol | 0.25 |
| Creamy cosmetic (II) -7 | - | Phenoxyethanol | 0.50 |
| Creamy cosmetic (II)-8 | - | n-hexyl glyceryl ether | 0.50 |

Next, the creamy cosmetics described above were subjected to storage stability tests. Each of the obtained creamy cosmetics (II)-1 to (II)-8 was placed in a polyethylene container having a diameter of 4 cm and a height of 6 cm and then subjected to a storage stability test at 50°C for one month. The evaluation method involved visual confirmation according to the criteria below. The results are shown in Table 6.

### [Evaluation method]

A smooth creamy state was maintained with no separation or sedimentation: A
Less than 5 mm of separation or sedimentation of aqueous components was observed: B
At least 5 mm but less than 1 cm of separation or sedimentation of aqueous components was observed: C
At least 1 cm of separation or sedimentation of aqueous components was observed: D

**Table 6**

| | Cosmetic used | Storage stability test evaluation result |
|---|---|---|
| Example 13 | Creamy cosmetic (II)-1 | A |
| Example 14 | Creamy cosmetic (II) -2 | A |
| Example 15 | Creamy cosmetic (II) -3 | A |
| Example 16 | Creamy cosmetic (II) -4 | A |
| Comparative Example 9 | Creamy cosmetic (II)-5 | D |
| Comparative Example 10 | Creamy cosmetic (II) -6 | D |
| Comparative Example 11 | Creamy cosmetic (II)-7 | C |
| Comparative Example 12 | Creamy cosmetic (II)-8 | D |

The results show that Examples 13 to 16, which used the method of this invention, exhibited extremely good storage stability. Therefore, this shows that by using this invention, it is possible to obtain a cosmetic that exhibits high storage stability in high temperature environments in summer etc.

### Industrial Applicability

The use of this invention can maintain good storage stability of a cosmetic by blending a compound represented by general formula (1), which exhibits an antibacterial/antiseptic effect and causes little skin irritation. Furthermore, even in cases where a commonly used antibacterial/antiseptic agent must be used, the use of this invention is extremely useful because it is possible to suppress a deterioration in storage stability caused by the commonly used antibacterial/antiseptic agent by using this invention.

## Claims

1. Use of a compound represented by the following general formula (1) for improving the storage stability of a cosmetic, wherein the cosmetic contains an antibacterial/antiseptic agent, and wherein the antibacterial/antiseptic agent is one or more types selected from the group consisting of phenoxyethanol, n-hexyl glyceryl ether, caprylyl glycol and ethylhexylglycerin; 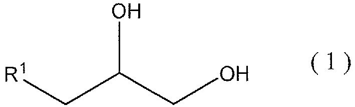 wherein, R¹ represents a group represented by general formula (2), 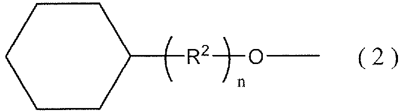 wherein, R² represents an alkylene group having 1 to 3 carbon atoms, and n represents an integer of 0 or 1.

2. The use according to claim 1, wherein the compound represented by general formula (1) is blended at an amount of 0.05 to 5 mass% relative to the total amount of a cosmetic.

3. The use according to claim 1 or claim 2, wherein the cosmetic contains a higher alcohol.

4. The use according to any one of claims 1 to 3, wherein the cosmetic contains a surfactant.

5. The use according to any one of claims 1 to 4, wherein the cosmetic is a creamy cosmetic.

## Patentansprüche

1. Verwendung einer Verbindung, die durch die folgende allgemeine Formel (1) wiedergegeben wird, für das Verbessern der Lagerstabilität eines Kosmetikums, wobei das Kosmetikum ein antibakterielles/antiseptisches Mittel enthält, und wobei das antibakterielle/antiseptische Mittel von einem oder mehreren Typen ist, die aus der Gruppe ausgewählt sind, die aus Phenoxyethanol, n-Hexylglycerylether, Caprylylglycol und Ethylhexylglycerin besteht, 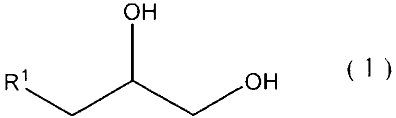 wobei R¹ eine durch die allgemeine Formel (2) wiedergegebene Gruppe wiedergibt, 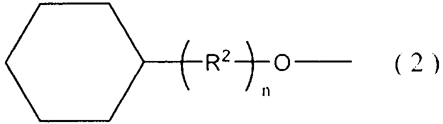 wobei R² eine Alkylen-Gruppe mit 1 bis 3 Kohlenstoffatomen wiedergibt und n eine Ganzzahl von 0 oder 1 wiedergibt.

2. Verwendung nach Anspruch 1, wobei die durch die allgemeine Formel (1) wiedergegebene Verbindung mit einem Anteil von 0,05 bis 5 Massen-% relativ zu der Gesamtmenge eines Kosmetikums beigemischt wird.

3. Verwendung nach Anspruch 1 oder 2, wobei das Kosmetikum einen höheren Alkohol enthält.

4. Verwendung nach einem der Ansprüche 1 bis 3, wobei das Kosmetikum ein Tensid enthält.

5. Verwendung nach einem der Ansprüche 1 bis 4, wobei das Kosmetikum ein cremiges Kosmetikum ist.

## Revendications

1. Utilisation d'un composé représenté par la formule générale (1) suivante pour améliorer la stabilité au stockage d'un cosmétique, dans laquelle le cosmétique contient un agent antibactérien/antiseptique, et dans laquelle l'agent antibactérien/antiseptique est un ou plusieurs types choisis dans le groupe constitué par le phénoxyéthanol, l'éther de n-hexyle et de glycéryle, le caprylylglycol et l'éthylhexylglycérine ; 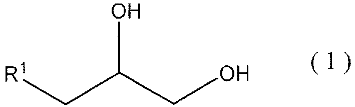 dans laquelle R¹ représente un groupement représenté par la formule générale (2), 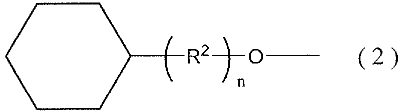 dans laquelle R² représente un groupement alkylène ayant 1 à 3 atomes de carbone, et n représente un entier de 0 ou de 1.

2. Utilisation selon la revendication 1, dans laquelle le composé représenté par la formule générale (1) est mélangé dans une quantité de 0,05 à 5 % en masse par rapport à la quantité totale d'un cosmétique.

3. Utilisation selon la revendication 1 ou la revendication 2, dans laquelle le cosmétique contient un alcool d'ordre supérieur.

4. Utilisation selon l'une quelconque des revendications 1 à 3, dans laquelle le cosmétique contient un tensioactif.

5. Utilisation selon l'une quelconque des revendications 1 à 4, dans laquelle le cosmétique est un cosmétique en crème.
